# EUROPEAN PATENT APPLICATION

(11) **EP 3 909 600 A1**
(43) Date of publication of application: **17.11.2021**
(21) Application number: 20174045.3
(22) Date of filing: 12.05.2020
(51) Int. Cl.: A61K 38/39, A61K 48/00, A61P 35/00, A61P 35/04, C07K 14/78

(54) **EMID2 PROTEIN AS ANTI-CANCER TREATMENT**

(71) Applicant: International Centre For Genetic Engineering And Biotechnology - ICGEB, 34149 Trieste (IT); Universita Degli Studi di Trieste, 34127 Trieste (IT)
(72) Inventor: ZACCHIGNA, Serena, 34151 Trieste (IT); GIACCA, Mauro, 34134 Trieste (IT); CAPPELLETTO, Ambra, 30029 San Stino di Livenza (VE) (IT)
(74) Representative: Sulcis, Roberta

(57) **Abstract**

The present invention refers to the EMID2 protein or a nucleic acid coding thereof for use for the treatment of a tumor or for the treatment or prevention of metastasis.

Compositions, vectors and formulations for the administration of EMID2 or a nucleic acid coding thereof for the above mentioned uses are also within the scope of the invention.

## Description

### FIELD OF THE INVENTION

The present invention relates to the pharmaceutical and medical fields.

In particular, the present invention refers to the EMID2 protein for the treatment of cancer and metastasis.

### BACKGROUND OF THE INVENTION

The heterogeneity of tumors from the same or from different anatomical locations is well-known to affect the development of an efficient cancer therapy.

Several studies demonstrated that cancer development is highly associated to the physiological state of the tumor microenvironment, that is characterized by common features, namely activation of fibroblasts, extracellular matrix (ECM) remodelling, increased hypoxia and altered angiogenesis that promote metastatasis. The tumor microenvironment profile is preponderant on prognosis and impacts efficacy of anti-cancer therapies.

Hence, a big effort has been made to develop new therapeutic strategies to modulate one or several features of the tumor microenvironment in order to block the metastatic invasion.

A treatment able to act on the tumor microenvironment thus disfavouring cancer cell growth and migration is highly desired.

Protein-based therapeutics have revolutionized the oncology space since they first appeared in the clinic two decades ago. Unlike traditional small-molecule chemotherapeutics, protein biologics are able to target molecules specifically associated with or overexpressed by tumors versus healthy tissue. Most of the existing protein-based biotherapeutics exert their effect by targeting receptors that stimulate their proliferation or blocking pathways that are used by cancer cells to avoid the immune response. While the first approved cancer biologics were monoclonal antibodies, the burgeoning field of protein engineering is spawning research on an expanded range of protein formats and modifications that allow tuning of properties such as target binding affinity, serum half-life, stability, and immunogenicity.

It has now been found that the administration of the protein EMID2 has a strong impact on several aspects of the tumor microenvironment.

Existing information on EMID2 is scanty. Gene association studies have associated it to ataxia and neurodegeneration [1], sight and hearing defects after Citalopram administration [2], behavioral defects after exposure to mercury [3]. Other studies have associated EMID2 to asthma and nasal polyps [4], which are characterized by abnormal secretion of extracellular matrix proteins. Indeed, another study indicates that EMID2 binds HSP47, a chaperone protein involved in collagen synthesis and expressed by nasal mucosa [5]. An additional study confirms a role of EMID2 in collagen fibrillogenesis in the cornea [6].

EMID2 belongs to the EDEN superfamily, which includes Emilin1 and Emilin2. These two proteins are characterized by the presence of an EMI domain at their N terminus, a C1q domain at their C terminus and a central super-coiled region. The same domain structure is common to Multimerin 1 and Multimerin 2, which also belong to the same superfamily. Instead, EMID2, similar to EMID1, is considered a member of the family because it contains the typical EMI domain, as well as a short super-coiled region, but it completely lacks the C1q domain, thus resulting much shorter than the other members. The EMI domain mediates important interactions of the protein. It inhibits TGFb maturation, leading to hypertension, as shown in Emilin1 knockout mice [7]. The EMI domain of Emilin3 binds heparin and heparan sulfates abundantly present in the extracellular matrix [8].

US20190241633 relates to the administration of RNA as a medicament; among the proteins for which RNAs can code, EMID2 is cited. The use of EMID2 is indicated for myocardial infarction and asthma.

In US20090280491, US20170343553 and WO2014162008 EMID2 is cited among other genes or factors as part of methods regarding prediction of some tumor features.

Use of EMID2 as a therapeutic protein with anti-tumor effects was never disclosed.

### SUMMARY OF THE INVENTION

It is an object of the invention the protein EMID2 or a nucleic acid coding thereof for use for the treatment of a tumor.

It is a further object of the invention the protein EMID2 or a nucleic acid coding thereof for use for the treatment and/or prevention of metastasis.

It has been found that EMID2 is a biologic drug able to interfere with multiple features of tumor microenvironment (such as extracellular matrix composition and angiogenesis) and therefore is effective on metastatic dissemination, which is the real killer in cancer. Due to these newly discovered effects, EMID2 can be advantageously used for treating tumors and for preventing or reducing the metastatic dissemination, i.e. for the treatment or prevention of metastasis.

A method for treating cancer and/or metastasis comprising the administration of EMID2 or a nucleic acid coding thereof to a subject in need thereof is also within the scope of the invention.

Compositions, vectors and formulations for the administration of EMID2 or a nucleic acid coding thereof for the above mentioned uses are also within the scope of the invention.

### DESCRIPTION OF THE INVENTION

### Definitions

Within the meaning of the present invention, "COL26A1" is a synonym of EMID2, both as a protein and as a gene.

Within the meaning of the present invention, for "a nucleic acid coding for EMID2" it is intended a nucleic acid able to code for the protein EMID2, i.e. a nucleic acid that in a suitable system, such as in a cell, allows to obtain said protein. Such nucleic acid can be any kind of nucleic acid, in particular it can be a genomic gene, a DNA stretch, a recombinant DNA, a RNA or a cDNA.

Within the meaning of the present invention, for "cDNA" or "complementary DNA" it is intended DNA synthesized from a single-stranded RNA (e.g., messenger RNA (mRNA)) template. Usually, it is the portion of a gene coding for a protein.

Within the meaning of the present invention, metastasis is the cancer spread from an initial or primary site to a different or secondary site within the host's body. It is herein intended metastasis of a cancer.

Within the meaning of the present invention, tumor is a disease involving abnormal cell growth with the potential to invade or spread to other parts of the body. Cancer is a synonym.

### Figures

Figure 1. EMID2 has been selected using a library including more than 1100 secreted factors in a functional screening for identifying anti-invasive proteins.
Figure 2. AAV9-EMID2 reduces the growth of multiple cancer cell lines in vivo (shown for LG lung carcinoma cells).
Figure 3. AAV9-EMID2 reduces the number and size of metastatic foci in the lung upon intravenous injection of different cancer cell lines (shown for LG lung carcinoma cells).
Figure 4. AAV9-EMID2 modulates the composition of the extracellular matrix inside the tumor mass *in vivo,* resulting in a profound alteration of its composition (reduced fibronectin and collagen I content) and mechanical properties, associated to a less invasive phenotype .
Figure 5. AAV9-EMID2 reduced the activation of cancer-associated fibroblasts (CAFs) *in vivo.*
Figure 6. AAV9-EMID2 inhibits tumor-associated angiogenesis (area covered by CD31+ endothelial cells) *in vivo* and interferes with the developmental angiogenesis in mice.

Protein EMID2 is the Collagen alpha-1(XXVI) chain protein, also named COL26A1.

The sequence of the protein EMID2 is available in the state of the art with the ID Q91VF6 (UniProt database; www.uniprot.org; Database Release October 15, 2019).

The protein EMID2 is commercially available. Therefore, it can be easily obtained by the skilled person in the field.

The sequence of the gene coding for the protein EMID2 is available in the art with the GeneID 140709 (database GENE, NCBI, updated on 13-Mar-2020).

Any version of the protein EMID2 can be used for the uses of the present invention. In particular, any protein having the biological activity of the protein EMID2 can be used. More in particular, the human EMID2 and the murine EMID2 can be preferably used.

EMID2 was found able to reduce the growth of multiple cancer cell lines *in vivo* by >30%.

Indeed, according to the present invention, EMID2 is for the use for the treatment of a tumor.

In an embodiment, said tumor is selected from the group consisting of colorectal cancer, breast cancer, lung cancer, epithelial tumors and melanoma. Preferably it is an epithelial tumor or a melanoma.

Preferably, it is a tumor that grows locally and metastasizes at distance in preferred organs, including colorectal cancer, breast cancer, lung cancer and melanoma.

It has been found that EMID2 inhibits the migration of multiple cell types.

Also, EMID2 inhibits the number and size of metastatic foci in the lung upon intravenous injection of 2 different cancer cell lines.

EMID2 inhibits cancer-associated fibroblasts (CAF) activation *in vitro* and modulates the composition of the extracellular matrix inside the tumor mass *in vivo,* resulting in a profound alteration of its mechanical properties, associated to a less invasive phenotype.

Furthermore, it exerts anti-angiogenic effects, inhibits tumor-associated angiogenesis *in vivo* and interferes with the normal development of the vasculature in neonatal mice, as well as with VEGF-induced angiogenesis in adult mice.

Therefore, according to the present invention, EMID2 can be advantageously used for the prevention or the treatment of metastasis.

In particular, it can be used in subjects at risk of developing metastasis, for example in subjects having tumors that have not yet colonized distant organs, or in subjects having tumors that already generated multiple metastases not suitable to surgical resection.

In embodiments of the present invention, EMID2 can be administered to a subject as a medicament by conventional methods.

For example, it can be administered in the form of recombinant protein.

A recombinant protein is obtained by isolation and cloning of the respective coding DNA (i.e. recombinant DNA) into an expression vector. Methods for obtaining recombinant proteins are well known in the art. Reference can be made for example to the following reviews: General introduction: recombinant protein production and purification of insoluble proteins. Ferrer-Miralles N, Saccardo P, Corchero JL, Xu Z, Garcia-Fruitós E.Methods Mol Biol. 2015; 1258:1-24; Recombinant protein production from stable mammalian cell lines and pools. Hacker DL, Balasubramanian S.Curr Opin Struct Biol. 2016 Jun;38:129-36.

The protein is preferably administered by parenteral or intravenous injection, but other forms are equally suitable for carrying out the present invention.

In an embodiment, EMID2 protein is administered by common ways of administration of collagenous proteins, such as intra-articular, cutaneous patches, oral supplements and intravenous injection.

The person skilled in the art will decide the effective time of administration, depending on the patient's conditions, degree of severity of the disease, response of the patient and any other clinical parameter within the general knowledge of this matter.

In other embodiments, a nucleic acid coding for the EMID2 protein is administered to a subject in need thereof.

Said nucleic acid can be for example a genomic gene, a DNA, a recombinant DNA, a RNA or a cDNA.

The nucleic acid is preferably administered in the form of complementary DNA (cDNA). Techniques for obtaining cDNA and for manufacturing and administering therapeutic genes are well known in the field.

Also said nucleic acid can be administered by means of a suitable vector known for the administration of genes.

A vector for use according to the present invention comprising at least a nucleic acid coding for the EMID2 protein is also an object of the invention.

A preferred vector is the adeno-associated vector (AAV) of any capsid serotype, either natural or artificial. AAV9 is a preferred vector.

Methods and formulations to administer a gene are conventional and well known in the art and do not need further explanations.

The use of a nucleic acid coding for EMID2 in gene therapy is also a preferred embodiment of the invention.

For gene therapy it is intended the therapeutic delivery of nucleic acid polymers into a patient's cells as a drug to treat a disease. According to the present invention, a nucleic acid coding for EMID2 can be delivered to cells of a subject in need thereof, for example a patient with a tumor.

Another object of the present invention is a pharmaceutical composition comprising the protein EMID2 or a nucleic acid coding thereof as active ingredient for the treatment of cancer or for the treatment and/or prevention of metastasis.

All these methods and formulations to administer a therapeutic protein are conventional and well known in the art and do not need further explanations.

In particular, the skilled person knows how to choose the suitable administration mode and vector, for example for human administration, according to the general knowledge in the field.

Reference can be made to Remington's Pharmaceutical Sciences, last edition.

The administration regime, dosage and posology will be determined by the physician according to his experience, the disease to be treated and the patient's conditions.

According to the administration route chosen, the compositions will be in solid or liquid form, suitable for oral, parenteral, intravenous or intra-arterial administration.

The compositions according to the present invention contain, along with the active ingredient, at least one pharmaceutically acceptable vehicle or excipient. These may be particularly useful formulation coadjuvants, e.g. solubilising agents, dispersing agents, suspension agents, and emulsifying agents.

Average quantities of the active agent may vary and in particular should be based upon the recommendations and prescription of a qualified physician.

Generally, the protein is administered in a "pharmaceutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. Generally, an effective dose will be from 0.01 mg/kg to 100 mg/kg, preferably 0.05 mg/kg to 50 mg/kg. Compositions may be administered individually to a patient or may be administered in combination with other agents, drugs, hormones, irradiation or surgery. For any compound, the therapeutically effective dose can be estimated initially either in cell culture assays or in animal models, usually mice, rats, guinea pigs, rabbits, dogs, monkeys or pigs. The same applies for administration of a nucleic acid coding for EMID2.

The following examples further illustrate the invention.

### EXAMPLES

### Example 1

We injected 24 pools of AAV9 vectors, each one containing an equimolar amount of 50 different secreted transgenes, bilaterally into the skeletal muscle of 3 month-old C57BL/6 mice (3 x 1010 viral genomes per muscle, 5 animals per pool). After 7 days, a time sufficient to drive robust transgene expression by AAV vectors, 3 animals per pool were injected into the same muscles with 50,000 lung cancer cells and sacrificed after additional 10 days. All the treated muscles were harvested for DNA extraction and quantification of each transgene by Next Generation Sequencing.

In this experimental system, cancer cells were expected to invade and progressively eliminate the surrounding muscle fibers, each one containing a few AAV genomes. If one of the transgenes inhibits the invasion by cancer cells, the producing muscle fiber will be protected and have a higher chance to survive compared to a fiber producing factors that either enhance or do not change the invasiveness of cancer cells. Thus, the AAV genomes within the muscle fibers that had resisted cancer cell invasion are expected to be enriched for transgenes encoding for protective proteins. To detect the factors carrying possible anti-invasive properties, we compared the enrichment of transgenes occurring in a transduced muscle in presence of cancer cells with the viral DNA recovered in muscles that received only the pool of AAV vectors.

By screening more than 1100 secreted factors of the library, we built a list of molecules and validated the 10 top hits for their ability to inhibit cell migration. Four out of 10 factors significantly reduced the migration of multiple cell types using different in vitro assays.

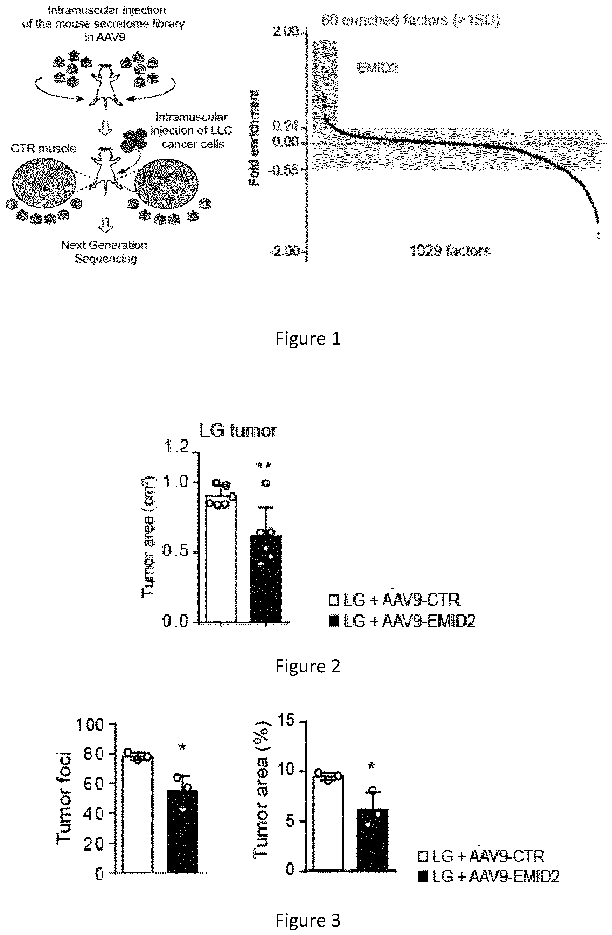

Therefore, we produced the AAV9 vector for their individual in vivo delivery and tested their capacity to inhibit tumor growth in a model of heterotopic tumor [9]. The most factor able to reduce tumor growth was EMID2 (Figure 1 and Figure 2).

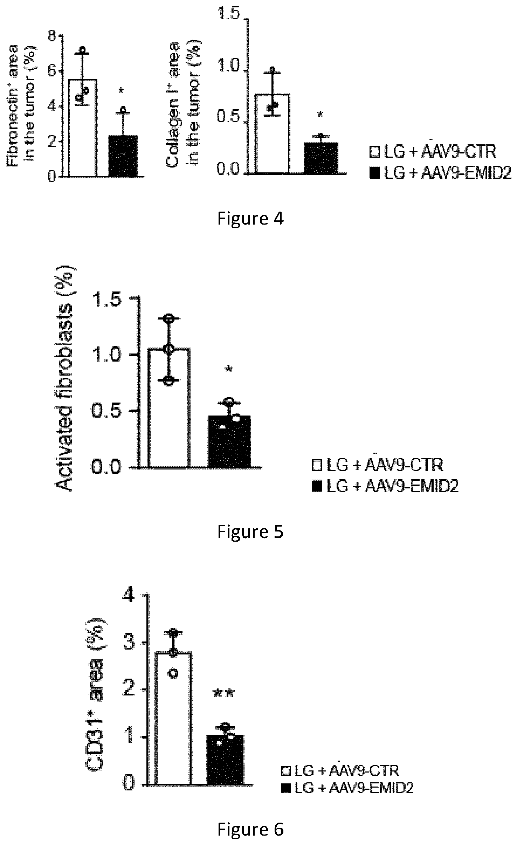

Further, we demonstrated that:
1) AAV9-EMID2 reduces the growth of multiple cancer cell lines *in vivo* by >30% (Figure 2).
2) AAV9-EMID2 inhibits tumor-associated angiogenesis *in vivo* and interferes with the normal development of the vasculature in neonatal mice (as well as with VEGF-induced angiogenesis in adult mice) (Figure 6)
3) AAV9-EMID2 reduces the activation of cancer-associated fibroblasts (CAFs) in vivo (Figure 5)
4) AAV9-EMID2 modulates the composition of the extracellular matrix inside the tumor mass in vivo, resulting in a profound alteration of its mechanical properties (associated to a less invasive phenotype) (Figure 4).
5) Mouse recombinant EMID2 inhibits the migration of multiple cell types, exerts anti-angiogenic effects and inhibits CAF activation in vitro.
6) The activity of EMID2 is largely mediated by its capacity to inhibit the maturation of TGFβ both in vivo and in vitro.
7) AAV9-EMID2 inhibits the number and size of metastatic foci in the lung upon intravenous injection of 2 different cancer cell lines (Figure 3)

Based on our results, the administration of the recombinant EMID2 has a strong impact on several aspects of the tumor microenvironment, thus disfavouring cancer cell growth and migration.

### References

1. Lim, J., et al., A protein-protein interaction network for human inherited ataxias and disorders of Purkinje cell degeneration. Cell, 2006. 125(4): p. 801-14.
2. Adkins, D.E., et al., Genome-wide pharmacogenomic study of citalopram-induced side effects in STAR*D. Transl Psychiatry, 2012. 2: p. e129.
3. Maccani, J.Z., et al., Placental DNA Methylation Related to Both Infant Toenail Mercury and Adverse Neurobehavioral Outcomes. Environ Health Perspect, 2015. 123(7): p. 723-9.
4. Pasaje, C.F., et al., Possible role of EMID2 on nasal polyps pathogenesis in Korean asthma patients. BMC Med Genet, 2012. 13: p. 2.
5. Sato, K., et al., Type XXVI collagen, a new member of the collagen family, is specifically expressed in the testis and ovary. J Biol Chem, 2002. 277(40): p. 37678-84.
6. Rada, J.A., P.K. Cornuet, and J.R. Hassell, Regulation of corneal collagen fibrillogenesis in vitro by corneal proteoglycan (lumican and decorin) core proteins. Exp Eye Res, 1993. 56(6): p. 635-48.
7. Zacchigna, L., et al., Emilinl links TGF-beta maturation to blood pressure homeostasis. Cell, 2006. 124(5): p. 929-42.
8. Schiavinato, A., et al., EMILIN-3, peculiar member of elastin microfibril interface-located protein (EMILIN) family, has distinct expression pattern, forms oligomeric assemblies, and serves as transforming growth factor beta (TGF-beta) antagonist. J Biol Chem, 2012. 287(14): p. 11498-515.
9. Carrer, A., et al., Neuropilin-1 identifies a subset of bone marrow Gr1-monocytes that can induce tumor vessel normalization and inhibit tumor growth. Cancer Res, 2012. 72(24): p. 6371-81.

## Claims

1. Protein EMID2 or a nucleic acid coding thereof for use for the treatment of a tumor.

2. The protein or the nucleic acid for the use of claim 1 wherein said tumor is selected from the group consisting of epithelial tumor, melanoma, colorectal cancer, breast cancer and lung cancer.

3. Protein EMID2 or a nucleic acid coding thereof for use for the treatment and/or prevention of metastasis.

4. The protein or the nucleic acid for the use of claim 3 wherein said protein or nucleic acid is administered to a subject at risk of developing metastasis.

5. The protein or the nucleic acid for the use of claim 3 or 4, wherein said protein or nucleic acid is administered to a subject having a tumor that has not yet colonized distant organs, or to a subject having a tumor that already generated multiple metastases not suitable to surgical resection.

6. The protein for the use of anyone of the preceding claims, which is a human protein or a murine protein.

7. The protein for the use of anyone of the preceding claims, which is administered in the form of a recombinant protein.

8. The protein for the use of anyone of the preceding claims, which is administered by intra-articular injection, cutaneous patches, oral supplement or intravenous injection.

9. The nucleic acid for the use of anyone of claims 1-5 which is a genomic gene, a DNA stretch, a recombinant DNA, a RNA or a cDNA, preferably a cDNA.

10. A vector comprising at least a nucleic acid coding for the EMID2 protein for use for the treatment of a tumor.

11. A vector comprising at least a nucleic acid coding for the EMID2 protein for use for the prevention and/or treatment of metastasis

12. The vector for the use of claim 10 or 11 which is an adeno-associated vector (AAV) of any capsid serotype, either natural or artificial, preferably the AAV9 vector.

13. A nucleic acid coding for the protein EMID2 for use in gene therapy for the treatment of a tumor or of a metastasis.

14. A pharmaceutical composition comprising the protein EMID2 or a nucleic acid coding thereof as active ingredient for use for the treatment of cancer.

15. A pharmaceutical composition comprising the protein EMID2 or a nucleic acid coding thereof as active ingredient for use for the treatment and/or prevention of metastasis.
